# EUROPEAN PATENT SPECIFICATION

(11) **EP 1 284 750 B1**
(45) Date of publication and mention of the grant of the patent: **24.01.2007**
(21) Application number: 01927508.0
(22) Date of filing: 03.05.2001
(51) Int. Cl.: A61K 38/48, A61K 38/47, A61K 31/355, A61P 19/04

(54) **PHARMACEUTICAL COMPOSITION**
PHARMAZEUTISCHE ZUSAMMENSETZUNG
COMPOSITION PHARMACEUTIQUE

(30) Priority: 04.05.2000 BR 0006719
(43) Date of publication of application: 26.02.2003
(73) Proprietor: Ribeiro Santana, Cristiano Alberto, 01250-000 Sao Paulo (BR)
(72) Inventor: Ribeiro Santana, Cristiano Alberto, 01250-000 Sao Paulo (BR)
(74) Representative: Hengelhaupt, Jürgen
(86) International application number: PCT/BR2001/000056
(87) International publication number: WO 2001/082956

(56) References cited:
- WO-A1-01/60399
- WO-A1-94/09816
- US-A- 4 678 668
- US-A- 5 516 517
- US-A1- 2001 047 037
- DATABASE WPI Week 200031, Derwent Publications Ltd., London, GB; AN 2000-351001, XP002957076 & BR 9 801 985 A (RIBEIRO SANTANA C A) 08 February 2000

## Description

The present invention refers to the use of more than 0.01% papain for the production of a pharmaceutical composition for treating collagenosis, such as keloids, hypertrophic scars, vasculophatic dermopaniculosis and the Dupuytren disease. The pharmaceutical composition is for topical application, non-toxic, featuring debridant and anti-inflammatory action with a high penetration rate through the skin. The pharmaceutical composition is for topical application, non-toxic, featuring debridant and anti-inflammatory action with a high penetration rate through the skin.

### PRIOR ART

BR 9 801 985 discloses a cream comprising 200 mg Vitamin E, 2% papain and 300 utr hyaluronidase for the treatment of Peyronie's disease. US 4 678 668 discloses compositions comprising 0.5-1.5 wt % papain and hyaluronidase for reducing soft tissue swelling and pain.

### DESCRIPTION OF THE INVENTION:

The skin permeability varies according to the region of the body, being the skin folds and the face those that present the highest absorption rate. A product applied over the skin will present a longer period of contact and percutanial absorption.

According to the classic book "Histologia dos epitélios", by Walter A. Hadler and Sineli R. Silveira, Editora Campus, Campinas, 1993, it is considered that: "bearing in mind the general morphological characteristics and the specialized functions that they perform, the epithelium cells are predominantly classified into two categories, which correspond to two epithelium classes: coating epithelium cells and secreting epithelium cells. The cells of these two classes mix with each other to constitute, respectively, the coating epithelium and the secreting epithelium, each one of them performing specific functions that are inherent to them. Such division is also fundamented in the distribution of these two classes of epithelium in the organism, which although wide is distinctive for both. With the purpose of forming the coating epitheliums the epithelium cells associate side-by-side, so as to originate "membranes" or layers superimposed over the base membrane, which function is to coat surfaces. On the contrary, the secreting cells unite to form organized functional units, better suited for performing their specialized function, related to the secretion products synthesis; thus are constituted the secreting units. The coating epitheliums are defined as living membranes, usually featuring a discontinuity, that isolate the organism from the environment, separating the internal media from the external one. Furthermore, these epitheliums isolate from each other the various internal media compartments, among which are the intravascular compartment, the serum compartment and several others. Among the various functions performed by the coating epitheliums some are performed by specialized variants that are specifically adapted to perform one or more functions. Others are incorporated as general functions presented without distinction by every coating epithelium cell. The coating epithelium cell, in the same way as most of the living cells, passively absorbs water and electrolytes and eliminates them actively; this function is well developed in the epithelium cells. On that account it is very important to observe that generally it is understood as absorption the penetration of solutions through the cells plasmatic membrane. However two different specific forms of absorption must be distinguished from one another: the passive absorption, that occurs according to the osmotic laws, and the active absorption, that entails the effective participation of the epithelium cell and that does not follow such physic laws. On the other hand it must be considered that every single substance that penetrates the interior of a multi-cellular organism, or else is excreted or eliminated, must cross at least one coating epithelium, because every superior organism is penetrated internally and externally by epitheliums. It must also be observed that the coating epitheliums, although continuously covering and protecting those surfaces it coats, are not impervious at all; that is why they do not behave as inert "membranes". On the contrary, they allow for the exchange of gases, water, several kinds of electrolytes and certain other solutes between the internal and the external media, or between the various internal compartments, which characterizes its permeability. The coating epithelium cells limit in a controlled and selective way the permeability of the respective epitheliums, with the purpose of protecting the organism and still participate of the control of its homeostasis. In order to perform such function the epitheliums are organized and arrange their cells in a special form, in order to build up coatings which cells abut the base membrane and are united with each other by means of intracellular junctions; in turn the cells are coated by the plasmatic membrane, which features special characteristics, and by the glicochalice, both able to express well defined functional properties. The functional characteristics expressed by the plasmatic membrane portion that coats the cells apical surface are different from those expressed by the portion situated in its basal or basolateral face; such differences, which occur mainly on the functional aspect, contribute for the remarkable degree of polarization expressed by the coating epithelium cells. The prime function performed by the coating epitheliums correspond essentially to the protection rendered to the surface that they coat, characterizing their protective coating function. Such function features a special characteristic, being a coating that, besides offering mechanical, physical and chemical protection to the coated surface, is not inert. The coating epitheliums are pervious, which allows for the controlled and selective passage of several products through its wall. There are many evidences in favor of the idea that the coating epitheliums permeability constitutes a fundamental property, with significant functional expression, for it is essential for the performance of several functions featured by the epitheliums, even more so because it is selective and its permeability degree presents a wide variation. It is fairly well demonstrated that the permeability degree influences strongly the function performed by the coating epitheliums:
1) wide permeability;
2) reduced permeability and
3) absence of permeability.

When there is a wide permeability, the epitheliums allow intense metabolic exchanges through their walls, with poor control and selectivity of its permeability. In these circumstances the epithelium acts on the filtration and transfer of metabolytes, these functions requiring little qualitative control; the exercise of these functions is subordinated to the epithelium intrinsic structure, which is adapted to act, mainly passively, being low the level of selective permeability. The coating epitheliums with a reduced degree of permeability, due to the characteristic that is so peculiar to them, present the property of partially controlling their own permeability, and above all their selectivity. As a consequence, these coating epitheliums present selective permeability, which allows them to interfere and qualitatively control their functional activity, as well as making them more able to actuate over the homeostasis control. The absence of epithelium permeability is correlated to the complex isolation of the coated surface and, on the other hand, to the better controlling of this epithelium function, because its cells, although very poorly pervious, present selective permeability. In this case the coated surface has its boundaries limited by a "membrane" impervious or very poorly pervious and very effective, that performs an important protective function, for it is able to discriminate exactly what can cross the epithelium. The coating epitheliums permeability is such an expressive functional property that it has been used as an important classification criterion to rank them in three classes:
1) pervious epitheliums;
2) poorly pervious epitheliums and
3) impervious epitheliums.

Because of their selective permeability, even in the inferior animals the epitheliums have assumed the function of coating the organism, constituting its external coating, with limiting and protective properties, not only morphological but also functional. Their cells, in principle very similar, behaved as a semi-pervious "membrane" poorly effective that acted passively, but which function allowed the separation, tough precarious and more morphological than functional, between the internal and the external media. It seem to be that the majority of the coating epitheliums acts as a barrier that prevents the free passive diffusion, because their permeability, which is selective, is conditioned to several factors among which stands out the electric potential present in their cells plasmatic membrane. The continuity of the epithelium coating is established as much through the intimate abutment of adjacent cells as through the presence of intercellular union devices. The epithelium cells are enveloped by the glicochalice, that also takes part of the coating function performed by the epithelium, in addition to aid the union between adjacent cells, because the intracellular adhesive is formed also by the glicochalice. Several experimental investigations confirm that the coating epitheliums selective permeability is associated to other specific functions expressed by their cells, namely: absorption, excretion and secretion. These functions, beyond their permeability, which constitutes their prime function, are responsible by the general functioning of the epithelium cell. The general functions performed by the coating epitheliums are basically the following:
1) surfaces protective coating function;
2) isolation and functional individualization of the internal media and of its distinct compartments, due to their cells selective permeability;
3) controlling the homeostasis of the internal medium and its compartments due to their cells ability to interfere in the epithelium selective permeability; the epithelium cells manifest the capacity to effect the absorption, secretion and excretion; such functions interfere on the epithelium permeability;
4) performance of the metabolic functions due to their ability to effect hydrosalinic exchanges and to effect metabolytes transfers due to their cells and intracellular spaces high degree of poorly selective permeability;
5) transport of products along the epithelial surface due to the participation of the cilia;
6) sensorial perception and
7) germinative function.

Among these functions, the first four derive mostly from the epithelium cells selective permeability, over which are additionally superimposed the additional affects corresponding to their properties of absorption; excretion and secretion. Among the general functions performed by the coating epitheliums, the selective permeability is responsible by the efficiency regarding the ability to coat, protect and isolate the surfaces, as well as to effect the control of the homeostasis; the passive absorption and the metabolytes transfer capacity are executed normally by the majority of the cells of these epitheliums, which demand only minor adaptations to become able to effectively perform such functions. On the contrary, the functions of absorption, excretion and secretion depend of properties that develop successively and would become paramount, mostly in some specialized types of coating epithelium, which adapted following a new and specific direction. The sensorial perception and the germinative function are more specific functions that are only manifest by certain epitheliums even more specialized. Considering their cell's morphological characteristics, the coating epitheliums have been classified according to the same number of cellular extracts they bear in: simple (a single extract) and stratified (two or more extracts). Both the simple epitheliums and the stratified ones, conforming to their cells format, are in turn subdivided into pavimentous, cubic or prismatic. The simple epitheliums are usually adapted to manifest wholly their most expressive fundamental property that consists in their permeability, which degree and selectivity vary. The simple coating epitheliums, constituted by a single layer of pavimentous or cubic-prismatic cells, present major differences regarding their functional properties, correlated not only to their cell's morphology, but also to the intracellular space's properties.. The simple pavimentous epitheliums are usually very pervious; the cubic-prismatic ones are less pervious. The coating epitheliums permeability, in addition to being selective, is controlled by their cell's functional activity, although the control looses efficiency in the same order as the intracellular space's permeability increases. The cubic-prismatic epitheliums, being less pervious than the pavimentous, are more effective to control their permeability. Based on the format of the epithelium cell, in its permeability and the coating epitheliums most common adaptations, it is possible to generate a provisional classification for these epitheliums. Thus, the simple coating epitheliums are divided into two classes: pavimentous and cubic-prismatic. Each class is subdivided according to its functional properties in open or pervious epitheliums, in semi-occlusive or poorly pervious and occlusive or impervious. In the simple coating epitheliums classification, the cubic epitheliums and the prismatic epitheliums are usually considered distinct, being defined and identified according to the format of the epithelium cells that make them up. However some functional studies have showed that the correlation between form and function presents several exceptions. For this reason a functional classification is adopted considering predominantly it's permeability. According to this criterion these epitheliums are denominated cubic-prismatic comprising the semi-occlusive and occlusive epitheliums. Following the same criterion the stratified epitheliums can be subdivided into: pavimentous and cubic-prismatic. The stratified epitheliums are adapted to perform primarily the mechanical protection function, because they are impervious or poorly pervious. The epitheliums comprise, in addition to the cells, the intercellular space and the base membrane, which interfere in their permeability degree; their permeability derives not only from their cell's peculiar properties, responsible for the transcellular permeability way, but also from the presence of another permeability way of their walls, constituting the intercellular or paracellular way. The transcellular way comprises two different ways that consist of the transmembranous way and the transcannular or trancitose way. It has been demonstrated, experimentally, that the coating epitheliums can be transposed by water and by substances of various natures, both through their epithelium cells (transcellular way) and through the way situated between their cells (intercellular way). In the first instance the epithelium cell can effect the permeab.ility control of the epithelium through its biological activity, making this process selective. As for the intercellular way permeability, the epithelium cell, although not behaving in a totally passive form, does not interfere directly in the transport selectivity. The sole form of cell active participation, in this instance, comprises the determination, exceptionally, the enlargement of the corresponding intercellular space. By means of the action of the microfilaments that constitute its cito-skeleton, the epithelium cell, specially those of certain types of simple coating epitheliums pavimentous of the open type, can change its format and retract segments of its cytoplasm; thus being able to influence the size of the intercellular space and regulate it. It has been established that the transcellular permeability of the simple coating epitheliums is perfectly distinct from the intercellular permeability, because both are subordinated to very different mechanisms. The epithelium cell permeability, which is selective, is influenced by its biological activity; on the contrary, the intercellular permeability is totally passive, and thus is not selective.

Several experimental results have confirmed that the transposition of solutions through the epitheliums is subject to multiple control mechanisms, among which is paramount the intrinsic functional activity of its cells. On the contrary, the intercellular space permeability is generally not controlled, because in this case the transposition of a molecule through the epithelium follows only the corresponding physical laws and is directly related to its diameter, its electrical cargo and, obviously, to the intercellular space size; these three variables constitute the main limiting factors that interfere on the intercellular permeability of the simple coating epitheliums. The transcellular permeability of the simple coating epitheliums can be exercised through two distinct and independent ways: the transmembrane way, which is the true transcellular way, and the transcannular way, which happens through the vesicles and the cannules or tubes of the vesicle-cannular system, found inside the cytoplasm of many types of coating epithelium cells". Consequently, the.coating epitheliums are pervious, which allows the controlled and selective passage of various products through its wall. It is demonstrated that the permeability degree affects strongly the coating epitheliums function.

Three types of coating epitheliums are thus considered:
1- Of wide permeability;
2- Of reduced permeability;
3- Of null permeability.

The purpose is to prove through the formulation that there is an intense metabolic exchange demonstrating that the epithelium actuates on the transfer of metabolytes. This penetration of substances is complete and gradual and trespasses these epithelium layers until it penetrates the small blood vessels, reaching the circulatory current.

There is a description of the molecules to estimate the coating epitheliums permeability. Ex.: Hemoglobin, Ferritin, Lipoproteins and enzymes.

### MICROCIRCULATORY UNIT

The microcirculatory unit, qualified as rotative plate of the cellular life, is a center of equilibrium of tissues, therefore the various vascular systems must adapt to the circulatory variations.

When the venous perturbation compensation mechanisms are overcome, the vascular and tissue structures change.

The venous stasis causes an increase in the intracapillar pressure. It generates an increase in the capillar permeability, which translates into the outflow of liquids and proteins of high molecular weight towards the conjunctive tissue.

The excess of permeability and the interstitial flooding originate a lymphatic overload, which causes an edema.

The liberation of aggressive substances, such as histamine, serotonine and prostaglandines unchain a series of tissue reactions. If the protein excesses are not depolymerized by the macrophages, there occurs a fibroblasts stimulation and the installation of fibrosis, which in turn keeps and makes worse the a venocapillar-limphatic stasis closing down the pathologic circle.

### ADIPOUS CELLULITIC TISSUE

The adipose tissue evolves slowly towards cellulites in four successive phases:
Interstitial Edema: a consequence of the venous stasis and of excessive capillar permeability, featuring capillar distension, increase in the passage of liquids and the appearance of edemas: in the conjunctive tissue core with lymphatic overload. The adipocytes get hypertrophied and bound together in a block.
Formation of a Conjunctive Network: the physic-chemical changes cause the formation of a network that infiltrates in escleroialine bands around the fat masses.
Formation of Micro-nodules, Later Macro-nodules: the adipose masses group up in closed micro-nodules in the conjunctive fiber and end up forming macro-nodules that can be identified through palpation.
Capillary Changes: are the same usually observed over the evolution of the varicous disease; ectasies, aneurysm, thickening of the basal layer.

Through the studies carried on by the applicant of the present invention, special importance is given to experimental research on animals and humans. The animals selected were adult rats (*Rattus Norvegicus Albinus*).

The histological cuts were analyzed under a common optical microscope and the results were submitted to a comparative analysis in all the groups, according to the classification of fibroblasts, collagenous fibers and leukocytes in specific tables for this purpose. Among adults were selected 21 women presenting multiple lesions of hypertrophic scars, keloids and vasculophatic dermopaniculosis (Cellulites). Six cases of hypertrophic scars, fifteen cases of cellulites and four cases of the Dupuytren disease were distributed.

**KINDS OF CELLULITIS**

| | |
|---|---|
| MILD CELLULITIS | 50% |
| HARD CELLULITIS | 20% |
| DERMATOUS CELLULITIS | 30% |

**AGE RANGE STUDIED:**

| | |
|---|---|
| 15 TO 25 YEARS | 40% |
| 26 TO 35 YEARS | 30% |
| 36 TO 45 YEARS | 19% |
| 46 TO 50 YEARS | 10% |
| 56 TO 65 YEARS | 06% |

### HYPERTROPHIC SCARS

In the four cases daily application twice a day.

The estimated improvement from 60 days onwards amounts to 30%, and in the beginning occurs the depigmentation and reduction in the height of the scar. After this period occurs the softening of the fibrous part of the lesion with a trend towards the re-epithelization of the affected area.

### DUPUYTREN DISEASE

The studies were conducted on the palm region, both in simple and multiple lesions, with the treatment being carried out in 1 or 2 hands simultaneously. The treatment conduct is to spread the gel twice a day over the lesion spot. The estimated improvement with absence of pain amounts to 40% from 30 days onwards.

After this period the fibrous hardened part (plaque) starts to soften.

The object of the present invention is the use of a **PHARMACEUTICAL COMPOSITION,** wherein said composition comprises:

| | |
|---|---|
| PAPAINE | more than 0.01% |

Advantageously the present invention may comprise the use of following formulation:

| | |
|---|---|
| PAPAINE | more than 0.01% |
| VITAMIN-E | from 10 to 2000mg |

Advantageously the present invention may provide the use of it's formulation:

| | |
|---|---|
| PAPAINE | more than 0.01% |
| HYALURONIDASE | 50 to 900 utr/mg |

More advantageously the present invention may comprise the use of following formulation:

| | |
|---|---|
| PAPAINE | more than 0.01% |
| VITAMIN-E | from 10 to 2000mg |
| HYALURONIDASE | 50 to 900 utr/mg |

The pharmaceutical composition is used mostly on colagenoses and fibrotic pathologies, being applicable in any form, specially gel, cream and cream gel, liquid, spray, aerosol and lyophilized.

## Claims

1. Use of more than 0.01 % Papain for the production of a pharmaceutical composition for treating collagenosis.

2. Use according to claim 1, whereby the composition comprises 50 to 900 turbidity reducing units (utr)/mg Hyaluronidase.

3. Use according to claim 1, whereby the composition comprises 10 to 2000 mg Vitamin E.

4. Use according to any one of claims 1 - 3, whereby the composition comprises more than 0.01 % Papain, 50 to 900 turbidity reducing units (utr)/mg Hyaluronidase and 10 to 2000 mg Vitamin E.

5. Use according to any one of claims 1 - 4, whereby the composition is presented in the form of gel, cream, cream-gel, aerosol, spray, liquid and lyophilized.

## Patentansprüche

1. Verwendung von mehr als 0,01% Papain für die Herstellung einer pharmazeutischen Zusammensetzung zur Behandlung von Kollagenose.

2. Verwendung nach Anspruch 1, wobei die Zusammensetzung 50 bis 900 TRU/mg (turbidity reducing units/mg) Hyaluronidase umfasst.

3. Verwendung nach Anspruch 1, wobei die Zusammensetzung 10 bis 2000 mg Vitamin E umfasst.

4. Verwendung nach einem der Ansprüche 1 bis 3, wobei die Zusammensetzung mehr als 0,01% Papain, 50 bis 900 TRU/mg Hyaluronidase und 10 bis 2000 mg Vitamin E umfasst.

5. Verwendung nach einem der Ansprüche 1 bis 4, wobei die Zusammensetzung in Form von Gel, Creme, Cremegel, Aerosol, Spray, Flüssigkeit und in lyophilisierter Form dargereicht wird.

## Revendications

1. Utilisation de plus de 0,01 % de papaïne pour la préparation d'une composition pharmaceutique destinée au traitement de la collagénose.

2. Utilisation selon la revendication 1, dans laquelle la composition comprend 50 à 900 TRU/mg (turbidity reducing units/mg) de hyaluronidase.

3. Utilisation selon la revendication 1, dans laquelle la composition comprend 10 à 2000 mg de vitamine E.

4. Utilisation selon l'une des revendications 1 à 3, dans laquelle la composition comprend plus de 0,01 % de papaïne, 50 à 900 TRU/mg de hyaluronidase et 10 à 2000 mg de vitamine E.

5. Utilisation selon l'une des revendications 1 à 4, dans laquelle la composition est présentée sous forme de gel, de crème, de gel-crème, d'aérosol, de spray, de liquide ou sous forme lyophilisée.
